# EUROPEAN PATENT APPLICATION

(11) **EP 2 845 544 A1**
(43) Date of publication of application: **11.03.2015**
(21) Application number: 14170878.4
(22) Date of filing: 03.06.2014
(51) Int. Cl.: A61B 17/02

(54) **Femoral elevator**

(30) Priority: 06.09.2013 US 201314020446
(71) Applicant: Symmetry Medical, Inc., Warsaw IN 46582 (US)
(72) Inventor: Bookwalter, John R., Putney, VT Vermont 05346 (US); Conley, Johnathon G., Silver Lake, IN Indiana 46982 (US); Gross, Jacob J., Winona Lake, IN Indiana 46590 (US); Kamel, Jason J. Kamel, Gardner, MA Massachusetts 01440 (US); Nordman, Mark A., Burket, Indiana 46508 (US); Parrott, Rebecca L., Claypool, IN Indiana 46510 (US); Whitehead, David J., Goshen, IN Indiana 46526 (US)
(74) Representative: Sawodny, Michael-Wolfgang

(57) **Abstract**

A bone elevator system includes: a bone hook (22); a first coupling device (20); an elevator arm member (16) including a substantially straight first elongate arm (120) and a substantially straight second elongate arm (122) which is offset from and fixedly coupled with the first elongate arm, the first coupling device coupling the first elongate arm with the bone hook, the second elongate arm configured for being coupled, by way of a second coupling device, with an anchoring post.

## Description

### BACKGROUND OF THE INVENTION

### 1. Field of the Invention

The present invention relates to orthopaedic devices, and, more particularly, to femoral elevators.

### 2. Description of the Related Art

Hip surgery can require using a support for a femur. Such a support is known which connects to a specialized surgical table.

What is needed in the art is a femoral elevator which is simple in design and that does not require a specialized surgical table.

### SUMMARY OF THE INVENTION

The present invention provides a femoral elevator that is simple in design and that does not require a specialized surgical table.

The invention in one form is directed to a bone elevator system which includes: a bone hook; a first coupling device; an elevator arm member including a substantially straight first elongate arm and a substantially straight second elongate arm which is offset from and fixedly coupled with the first elongate arm, the first coupling device coupling the first elongate arm with the bone hook, the second elongate arm configured for being coupled, by way of a second coupling device, with an anchoring post.

The invention in another form is directed to a method for using a bone elevator system, the method including: providing a bone hook, a first coupling device, and an elevator arm member including a substantially straight first elongate arm and a substantially straight second elongate arm which is offset from and fixedly coupled with the first elongate arm; coupling, using the first coupling device, the first elongate arm with the bone hook; coupling, using the second coupling device, the second elongate arm with an anchoring post.

An advantage of the present invention is that it provides a surgical instrument to be used in minimally invasive surgery hip arthroplasty procedure.

Another advantage of the present invention is that it provides a device for hip joint reconstruction that can optionally be used with an anterior surgical approach. The device is a hip femoral elevator which optionally can be used as an anterior approach hip femoral elevator. Advantages of the anterior approach include little detachment of muscle from bone (thereby decreasing recovering time and improving post-operative mobility) and a smaller incision. Disadvantages of the anterior approach include difficulty in exposing the proximal femur and a specialized surgical table is thereby recommended. Such a specialized surgical table can be the HANA® table. However, the present invention provides a device that facilitates exposure of the proximal femur without using a specialized surgical table, such as the HANA® table. The present invention can thus optionally be used with a standard surgical bed or table. Application of the present invention is not limited to the anterior surgical approach.

Yet another advantage of the present invention is that the L-shaped elevator arm member, together with the post coupling device, facilitate movement in all six degrees of freedom and provide an additional seven and one-half inches of extension above the table (the table on which the surgical patient lies).

Yet another advantage is that the bone hook coupling device and the bone hook of the bone elevator system of the present invention together have an "open" design so as to facilitate movement of the bone hook coupling device and the bone hook about and/or along the elevator arm member. The bone hook coupling device can be hooked onto the elevator arm member after the construct (including the post rail, the post coupling device, and the elevator arm member) is in position. Similarly, the bone hook can be attached to the bone hook coupling device after this construct is in position.

Yet another advantage is the bone elevator system of the present invention can be a femoral elevator. As such, the femoral elevator of the present invention is a reusable device that can be used to facilitate exposure of the femur during a surgical procedure. The femoral elevator can be attached to the rail of an operating room surgical bed/table.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a perspective view of the bone elevator system of the present invention;
Fig. 2 is a partially exploded, perspective view of the rail post of the bone elevator system of Fig. 1;
Fig. 3 is a cross-sectional view of the rail post of Fig. 1 taken along line 3-3 of Fig. 1;
Fig. 4 is an exploded, perspective view of the post coupling device of the bone elevator system of Fig. 1;
Fig. 5 is a side view of a passageway of the post coupling device of Fig. 4, with portions broken away;
Fig. 6 is an exploded, perspective view of the elevator bar member of the bone elevator system of the Fig. 1;
Fig. 7 is an exploded, perspective view of the bone hook coupling device of the bone elevator system of Fig. 1;
Fig. 8 is an exploded, perspective view of the bone hook coupling device of the bone elevator system of Fig. 1;
Fig. 9 is a cross-sectional, perspective view of the ratchet mechanism of the bone elevator system of Fig. 1 taken along line 9-9 of Fig. 7; and
Fig. 10 is an exploded, perspective view of the lateral retention ring assembly of the bone elevator system of Fig. 1.

Corresponding reference characters indicate corresponding parts throughout the several views. The exemplifications set out herein illustrate one embodiment of the invention, and such exemplifications are not to be construed as limiting the scope of the invention in any manner.

### DETAILED DESCRIPTION OF THE INVENTION

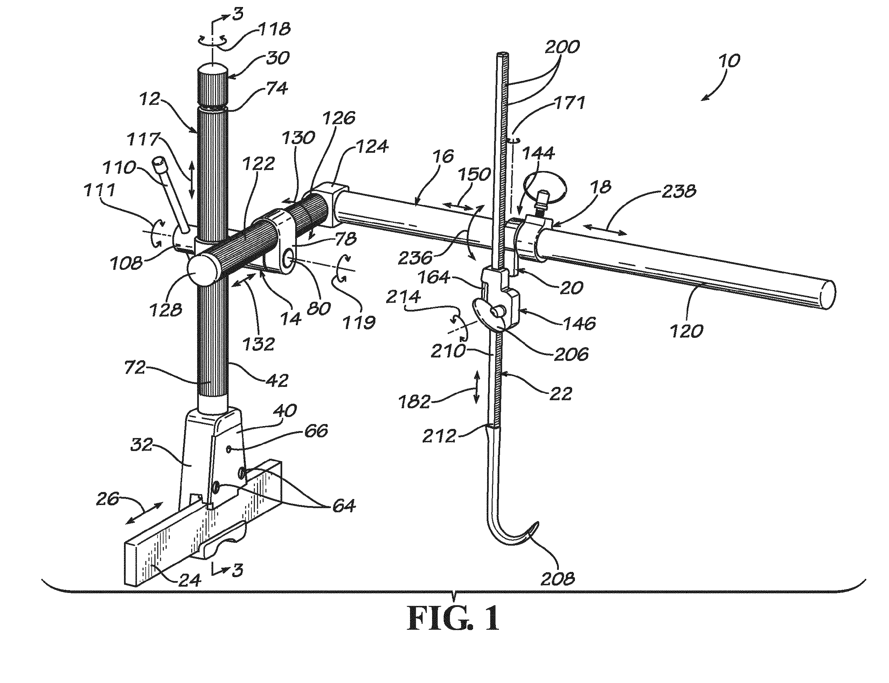

Referring now to the drawings, and more particularly to Fig. 1, there is shown a bone elevator system 10 which generally includes an anchoring post 12, a post coupling device 14, an elevator arm member 16, a lateral retention ring assembly 18, a bone hook coupling device 20, and a bone hook 22.

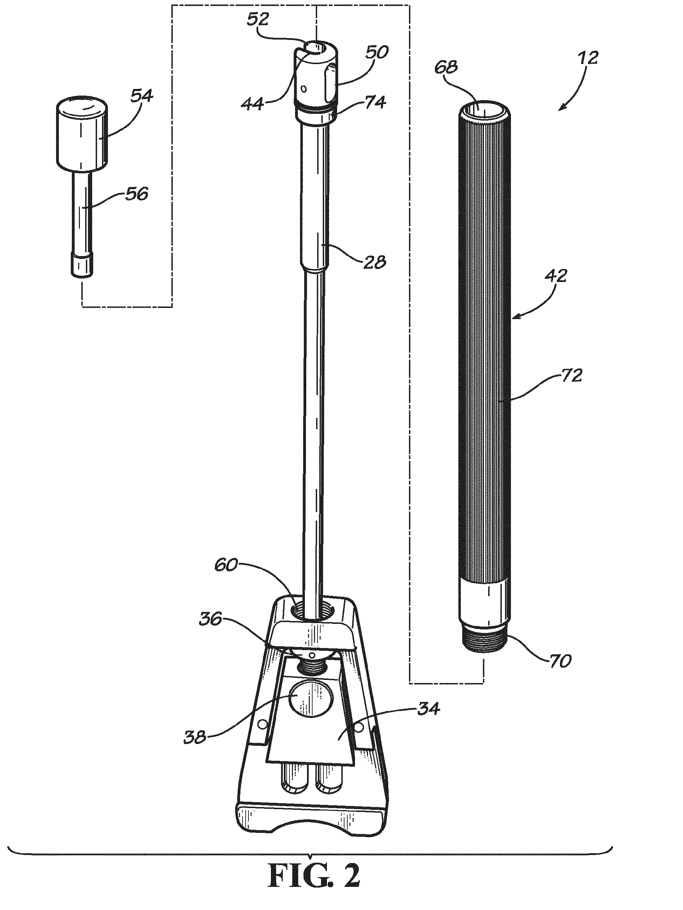

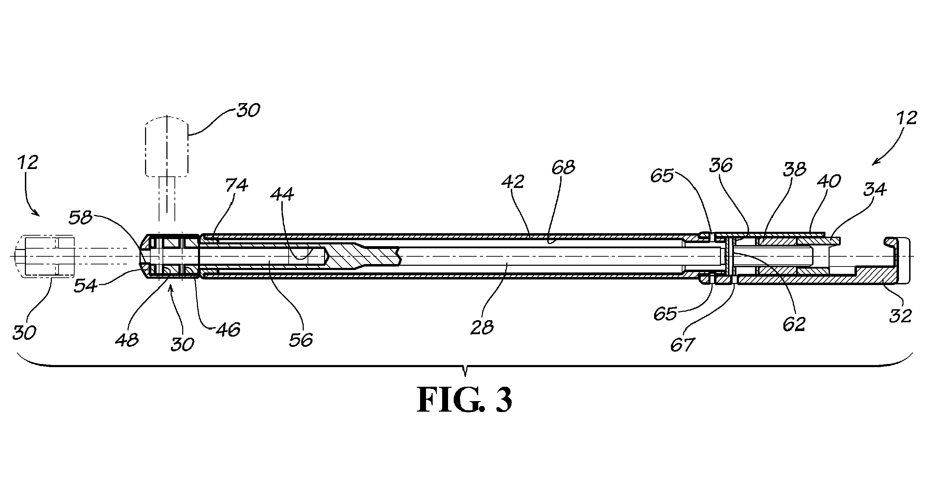

Figs. 1-3 show anchoring post 12. Anchoring post 12 serves to anchor and thereby support post coupling device 14, elevator arm member 16, lateral retention ring assembly 18, bone hook coupling device 20, and bone hook 22 to a support mechanism, such as a surgical bed or surgical table. According to one embodiment of the present invention, anchoring post 12 is a vertical rail post 12, as shown in the figures. Rail post 12 stands substantially vertically and is mounted to a rail 24, which can be a bed rail or a table rail. This bed or table is not shown in the drawings, but it is understood that rail 24 can extend substantially horizontally along the side of the table or bed on which a medical patient lies during a surgical procedure. Thus, rail post 12 is configured for coupling with rail 24. Rail post 12 is configured for sliding substantially horizontally along rail in either direction (as shown by double-arrow 26) and can be selectively positioned in a plurality of positions along rail. As shown in Figs. 2 and 3, rail post 12 can be a rail post assembly. As an assembly, rail post 12 can include a post rod 28, handle assembly 30, a clamp housing 32, a clamp 34, a locking nut 36, an adjustment nut 38, clamp cover 40, and a bar 42. Certain components (not necessarily all) of rail post 12 are known.

Post rod 28 is an elongate rod and has a top portion and a bottom portion. The top portion of post rod 28 includes an elongate blind hole 44, which can have a cone-shaped terminating end. The top portion further includes two opposing threaded holes each of which threadably receives a ball plunger 46. Ball plunger 46 can include a ball (which is biased outwardly) and a nylon locking insert (Nylok), and, in general, the ball plunger 46 can be made of 303 series stainless steel (such as part no. SSB-46P from Vlier Eng. Corp.); this is provided by way of example and not by way of limitation. Ball plungers 46 compress onto post rod 28 and thus provide some resistance to handle assembly 30 coming out of blind hole 44 of post rod 28, although a surgeon, for example, can readily move post rod 28 to overcome the compressive force of ball plungers 46. Ball plungers 46 can also provide a tactile sensation to the user, to include when the distal end of handle 56 (which forms a stepped portion of handle 56 and thus has a greater radius than other portions of handle 56) passes adjacent to or by ball plungers 46 during use of handle assembly 30). The top portion further includes two opposing holes each of which receives a pin 48 which serves as a handle stop (to stop handle 56). Thus, during use when a surgeon pulls cap assembly 30 up from hole 44, the stepped distal end of handle 56 is prevented from being fully withdrawn from post rod 28 by pins 48. Each pin 48 can be made of DIN 1.4301 or 1.4305 German stainless steel; this is provided by way of example and not by way of limitation. Pins 48 can be welded to post rod 28. Pins 48 can protrude (for example, approximately 0.25 mm) into the elongate blind bore 44 in two places and must clear the inside diameter of post rod 28 so as to serve as a handle stop. Thus, pins 48 captures handle 56 in the vertical direction relative to post rod 28. Communicating with the centrally located elongate blind hole 44 are opposing holes 50, 52 in post rod 28, hole 50 being elliptical, hole 52 also being elliptical but being open on top and not extending down as far as hole 50. The bottom portion of post rod 28 is threaded. Post rod 28 can be made of DIN 1.4305 German; this is provided by way of example and not by way of limitation.

Handle assembly 30 mounts to the upper portion of post rod 28 but can be detached therefrom. Handle assembly 30 includes a cap 54 and a handle 56. Cap 54 includes a hole 58 in which one end of handle 56 is attached and welded thereto; that is, at this end, there is a weld all around this end and hole 58 (and assembly of cap 54 and handle 56 includes grinding and polishing smooth to match radius of cap 54). Cap 54 can be made of DIN 1.4301 German stainless steel; this is provided by way of example and not by way of limitation. Handle 56 is an elongate rod. Handle 56 can be made of DIN 1.4301 German stainless steel; this is provided by way of example and not by way of limitation. During use, handle assembly 30 can be pulled partially out of blind hole 44, pins 48 preventing handle assembly from being pulled all of the way out from post rod 28. Further, holes 50 and 52 cooperate with handle 56 so that handle assembly 30 can be used as a turning mechanism to turn post rod 28, this turning of post rod 28 causing clamp 34 to clamp down onto rail 24 or to be released from rail 24 (depending upon the direction of turning). Fig. 3 shows schematically that handle assembly 30 can be pulled partially out of hole 44 (the schematic depiction of handle assembly 30 extending to the left of the primary drawing in Fig. 3. Then, handle assembly 30 can be rotated approximately ninety degrees in holes 50 and 52 so that handle assembly 30 can be used as a turning mechanism (as indicated by the schematic depiction of handle assembly 30 in Fig. 3 that is perpendicular to rail post 28). More specifically, the larger hole 50 provides clearance to allow the distal end of handle 56 to rotate clear of post rod 28, while the smaller hole 52 allows the portion of handle nearer to cap 54 to rest within hole 52 upon rotation of handle assembly 30 ninety degrees. These holes 50 and 52 thus capture handle 56 in a horizontal position with appropriate pressure by the user.

Clamp housing 32 includes a space that houses clamp 34, adjusting nut 38, and locking nut 36. Clamp housing 32 includes a threaded through-hole 60 which is threadably connected to the bottom of bar 42 (which can be referred to as a grooved support cover 42). Through-hole 60 also receives the bottom portion of post rod 28 therethrough. Clamp 34 can be made of AlMgSi 0.5 or AlMgSi 1 German Aluminum and can have a blue anodized finish; this is provided by way of example and not by way of limitation. Holes 65, provided in clamp housing 32, are 180° apart from one another. Additional holes (which are also 180° apart from one another and are comparable in diameter to holes 65) are provided in clamp housing 32 at ninety degrees to holes 65; these two additional holes are not shown in the figures. Thus, these four holes are spaced ninety degrees apart from one another about clamp housing 32. Four corresponding holes can also be drilled into bar 42 and threaded (these holes in clamp housing 32 can be drilled at the same time as the corresponding holes in bar 42, or they can be drilled separately). These four holes in clamp housing 32 and these four holes in bar 42 receive set screws (not shown in the figures) therein; that is, hole 65 which aligns with a single hole in bar 42 together threadably receive one set screw, the other aligned holes each receiving a single set screw as well. These four set screws can be made of M4 DIN 916 V2A, which is provided by way of example and not by way of limitation. These four set screws can be secured within these corresponding holes by Loctite epoxy (by applying the epoxy before and/or after threading the screws in the holes). These set screws serve to resist torque applied to bar 42 and thus provide a mechanical fixation of clamp housing 32 to bar 42.

Locking nut 36 is fixedly secured to a bottom portion of post rod 28 by way of a pin 62 (by way of example and not by way of limitation, this pin 62 can be made of DIN 1.4301 or 1.4305 German stainless steel). During assembly, using a pilot hole extending radially on locking nut (this pilot hole may be formed in only one wall of locking nut), a hole can be drilled through locking nut 36 and post rod 38, and pin 62 can be attached in this hole. Locking nut 36 can rotate within the space of clamp housing 32 and serves to keep the bottom portion of post rod 28 within the space of clamp housing 32 and can serve to prevent clamp 34 from moving too far up post rod 28 during rotation of post rod 28. Locking nut 36 can be made of CuSn6 or CuSn8 German bronze, and the finish can be chrome plated; this is provided by way of example and not by way of limitation. Adjustment nut 38 is positioned within a side-facing through-hole of clamp 34 and is threaded onto the bottom portion of post rod 28 below locking nut 36 (post rod 28 also extending through a top hole in clamp 34, this top hole and the side-facing through-hole of clamp 34 being in communication with one another). As post rod 28 is rotated by a user, adjustment nut 38 is caused to translate up and down (depending upon the direction of rotation of post rod 28) on the threaded portion of post rod 28. As adjustment nut 38 moves up and down on post rod 28, clamp 34 correspondingly moves up and down on post rod 28 and thereby can tighten onto or loosen from the horizontal rail 24, thereby selectively securing clamp 34 to rail 24 or releasing clamp 34 from rail 24. Adjustment nut 38 can be made of CuSn6 or CuSn8 German bronze; this is provided by way of example and not by way of limitation.

Clamp 34 moves up and down on post rod 28 by way of adjustment nut 38. Clamp 34, when in an engagement position, can contact a bed or table rail 24 and clamp the rail post 12 in position on the rail 24. Clamp 34 can be released from rail 24 so that rail post 12 can be moved to another position on rail 24. Clamp 34 can be made of 1.4305 German stainless steel and have a satin finish; this is provided by way of example and not by way of limitation.

Clamp cover 40 can be screwed to clamp housing 32 with two flathead screws 64 (by way of example and not by way of limitation, flathead screws 64 can be made of DIN 1.4301 or 1.4305 German stainless steel). Clamp cover 40 can be made of 1.4301 German stainless steel and can have a satin finish; this is provided by way of example and not by way of limitation. Clamp cover 64 can include a through-hole 66 which can be used to insert a lubricant.

Bar 42 can also be referred to as a grooved support cover or a sleeve. Bar 42 has a longitudinally extending passageway 68 therethrough and can thus be deemed to be hollow. Post rod 28 is positioned within this passageway 68. Bar 42 has an externally threaded section 70 on the bottom portion of bar 42. This threaded section 70 of bar 42 is threaded into the through-hole 60 of clamp housing 32 and is thereby affixed thereto. Bar 42 does not rotate with post rod 28. Bar 42 further includes a radially outwardly facing peripheral surface which has a plurality of grooves 72 running longitudinally (that is, axially) on bar 42. Each groove 72 can be spaced apart from an adjacent groove 72 by eight degrees (this is provided by way of example and not by way of limitation). Grooves 72 can be formed by a milling operation (where tool is moving and bar 42 remains constant). Grooves 72 provide positive engagement with post coupling device 14 while still allowing for adjustment at eight degree increments. A top portion of bar 42 can be positioned over a bushing 74. Bushing 74 can be formed generally as an annular ring. Bushing 74 can surround post rod 28. Bushing 74 can rotate with post rod 28, at least intermittently. Bar 42 can be made of DIN 1.4301 or 1.4305 German stainless steel; this is provided by way of example and not by way of limitation. Bushing 74 can be made of bronze, for example (but not by way of limitation), CuSn6 or CuSn8 German bronze. Bushing 74 can have a finish which is a nickel plate. This material and finish is provided by way of example and not by way of limitation.

Notwithstanding the disclosure above, the finish of rail post 12 (for example, individual parts) can optionally be glass bead or satin, and individual parts can be finished and passivated before assembly. The finish of clamp housing 32 can be blue anodized. The finish of locking nut 36 can be nickel plated. The pertinent components of rail post 12 can be lubricated before each use by way of hole 66 in cover plate 40. Lubrication and/or flushing can occur by hole 66 and/or hole 67. Cracks, pits, and visible imperfections are not permissible, and all burrs and sharp edges are to be removed, with respect to rail post 12. The rail post 12 shall pass test TM-120 boil test and STM-04 autoclave test. Further, it is noted that Fig. 3 shows additional structures not included or shown in Figs. 1 and 2.

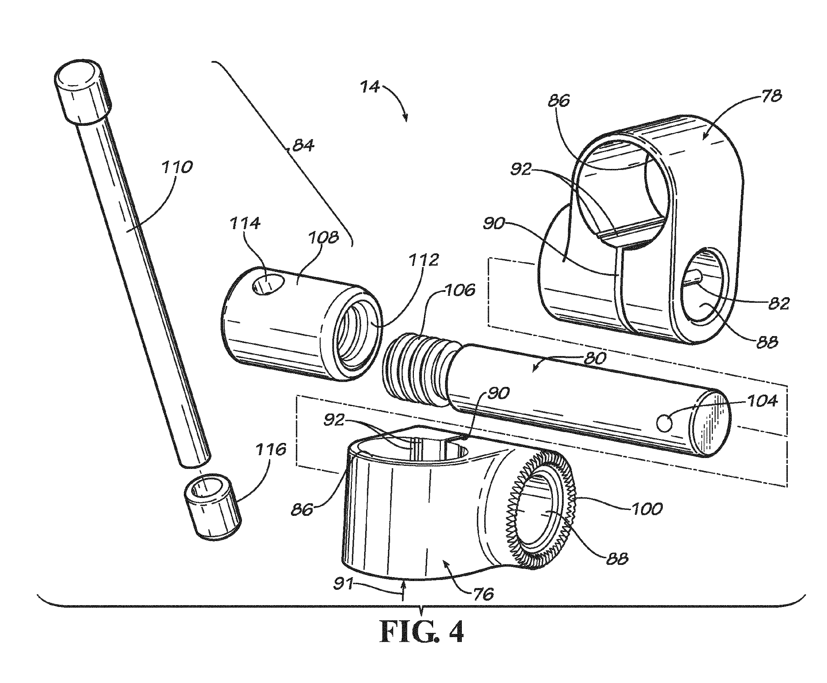

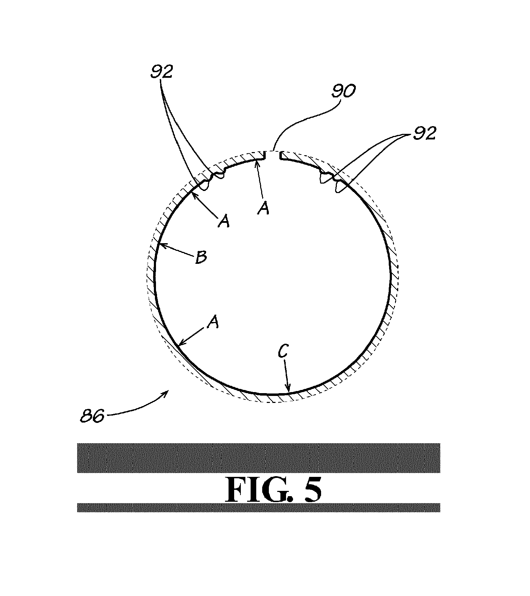

Figs. 4 and 5 show post coupling device 14. Post coupling device 14 couples rail post 12 with elevator arm member 16. Post coupling device 14 includes a first coupling half 76, a second coupling half 78, a center post 80, a pin 82, and a T-handle tightener 84. First coupling half 76 can be referred to as a lateral coupling half, considering that it is positioned to the lateral side of the surgical table or bed (and also to the patient). First coupling half 76 includes two passageways 86, 88 (first passageway 86 and second passageway 88) which are substantially perpendicular to one another. The first passageway 86 is formed by a split ring such that the two portions adjacent the split 90 can be brought closer together when post coupling device 14 is tightened or spread apart when post coupling device 14 is loosened (by way of tightener 84). An inner surface of first passageway 86 also includes a plurality of ridges 92 (such as two ridges 92 on each side of the split 90, but more or less ridges 92 can be provided); these ridges 92 are spaced apart so as to engage grooves 72 on the external surface of bar 42 and thereby prevent bar 42 and first coupling half 76 from rotating relative to one another when first coupling half 76 is tightened about bar 42. The second passageway 88 receives center post 80 therethrough. Further, on an outer axial end of second passageway 88 (the end facing second coupling half 78), the second passageway 88 includes a plurality of teeth 100, which are configured to engage grooves between a plurality of teeth 100 on second coupling half 78; this engagement relative to teeth 100 of both coupling halves 76, 78 helps to prevent first coupling half 76 and second coupling half 78 from rotating relative to one another when tightener 84 is tightened.

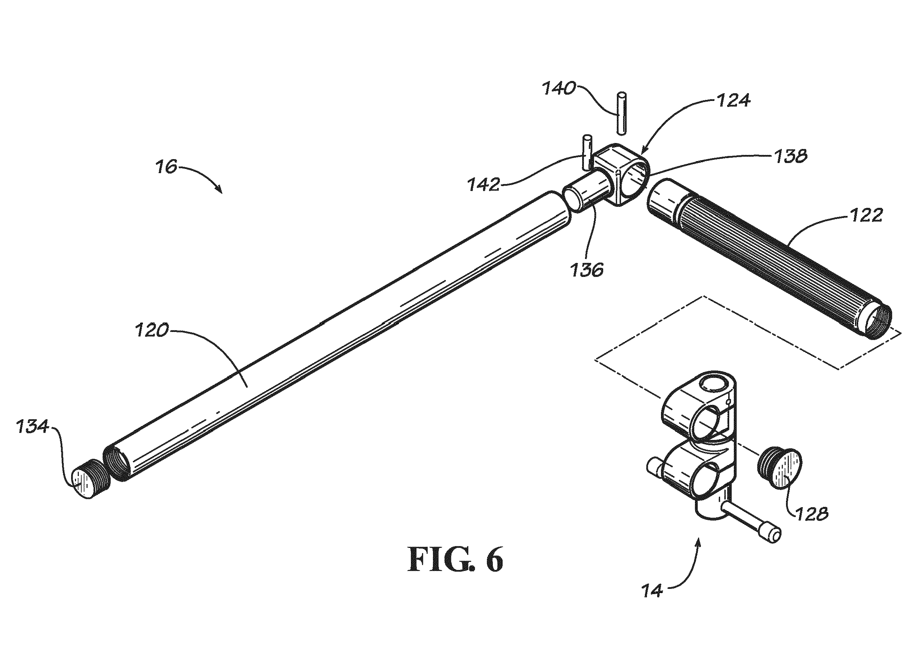

Second coupling half 78 includes two passageways 86, 88 (first passageway 86 and second passageway 88) which are substantially perpendicular to one another. The first passageway 86 is formed by a split ring such that the two portions adjacent the split 90 can be brought closer together when post coupling device 14 is tightened or spread apart when post coupling device 14 is loosened (by way of tightener 84). An inner surface of first passageway 86 also includes a plurality of ridges 92 (such as two ridges 92 on each side of the split 90, but more or less ridges 92 can be provided); these ridges 92 are spaced apart so as to engage grooves 126 on an external surface of short bar 122 and thereby prevent short bar 122 and second coupling half 78 from rotating relative to one another when second coupling half 78 is tightened about short bar 122. The second passageway 88 receives center post 80 therethrough. Pin 82 is inserted through opposing holes in second coupling half 78 (one of these holes is shown in Fig. 6, but it is understood that these holes can be smoothed over during manufacture) and also through the through-hole 104 near one longitudinal end of center post 80; in so doing, center post 80 cannot rotate relative to second coupling half 78 because pin 82 is welded on both sides of pin 82 to second coupling half 78. Further, on an outer axial end of second passageway 88 (the end facing laterally and thus towards first coupling half 76), the second passageway 88 includes a plurality of teeth 100, which are configured to engage grooves between the plurality of teeth 100 on first coupling half 76 so as to prevent first coupling half 76 and second coupling half 78 from rotating relative to one another when tightener 84 is turned so as to tighten post coupling device 14. First and second coupling halves 76, 78 can be made of 304 stainless steel adhering to ASTM F899, have a satin finish (excluding teeth side walls), and can be passivated (per ASTM A967); this is provided by way of example and not by way of limitation.

Fig. 5 shows a detail view of first passageway 86 of first coupling half 76, the detail view being taken generally in the direction of arrow 91 in Fig. 4. Fig. 5 focuses on showing the inner surface of first passageway 86. Arrows A, B, and C in Fig. 5 are associated with five arrows, these arrows pointing to different regions on the inner surface. Arrows A, B, and C are each associated with a region having a specific radius on the inner surface, arrow A being in a region having a radius A, arrow B being in a region having a radius B, and arrow C being in a region having a radius C. Radii A, B, and C are different from one another (the arrows with radius A have the same radius). The relative dimensions are as follows: A < B < C. Thus, these radii do not share a common center point. Regions with arrows A and B can be mirrored on the other side of the inner surface. More specifically, arrows A and B are shown in the portion of first passageway 86 ranging from 6 o'clock to 12 o'clock in Fig. 5; if the page of Fig. 5 were folded from 12 o'clock to 6 o'clock in Fig. 5, then the portion ranging from 12 o'clock to 6 o'clock would mirror the portion ranging from 6 o'clock to 12 o'clock. When first passageway 86 is in a relaxed position, first passageway 86 is thus not a circle. However, when first passageway 86 is compressed so as to substantially close split 90, first passageway 86 virtually forms a perfect circle, for purposes of gripping. First passageway 86 of second coupling half 78 is substantially similar to what is shown in Fig. 5.

Center post 80 is a longitudinally extending rod and includes a transverse through-hole 104 on one longitudinal end and a threaded portion 106 on the opposing longitudinal end. Center post 80 is inserted through each second passageway 88 of first coupling half 76 and second coupling half 78 and is positioned therein. The through-hole 104 receives pin 82 therethrough when pin 82 is inserted in corresponding through-holes in second coupling half 78. Center post can be made of 420 stainless steel adhering to ASTM F899, have a satin finish, can be passivated (per ASTM A967), and can be heat treated to Rc 49-54; this is provided by way of example and not by way of limitation. Cross pin 82 can be made of 416 stainless steel and can be passivated (per ASTM A967); this is provided by way of example and not by way of limitation.

Tightener 84 includes a threaded body 108 and a bar 110 (which can be referred to as a T-bar 110). Threaded body 108 includes a longitudinally extending blind hole 112, which can terminate in a coned section inside threaded body 108. Threaded body 108 includes threads and corresponding transverse through-holes 114 in the cylindrical wall of threaded body 108. Threaded body 108 is threadably secured to the threads 106 of center post 80. Threaded body 108 can be made of NITRONIC® 60 stainless steel adhering to ASTM A276 or ASTM F899, have a satin finish (excluding internal features), and can be passivated (per ASTM A967); this is provided by way of example and not by way of limitation. T-bar 110 can be made of 17-4 PH stainless steel adhering to ASTM A564 or ASTM F899, have a satin finish, can be passivated (per ASTM A967), and can be heat treated to condition H900, Rc 40 min ("minimum"); this is provided by way of example and not by way of limitation. Tightener 84 can be passivated per ASTM A967. An endcap 116 can be welded to T-bar 110 (such as, for example, by way of gas tungsten arc welding 308 or 308 L, with filler, and polished smooth). Endcap 116 can be made of 17-4 PH stainless steel adhering to ASTM A564 or ASTM F899, have a satin finish, can be passivated (per ASTM A967), and can be heat treated to condition H900, Rc 40 min; this is provided by way of example and not by way of limitation. Tightener 84 can be formed by milling and machining (such as wire electrical discharge machining (wire EDM)), holes 114 being formed by wire EDM; these manufacturing processes are provided by way of example and not by way of limitation. T-bar 110 is received in transverse through-holes 114 in threaded body 108 and is able to slide within holes 114. By turning T-bar 110, threaded body 108 is caused to move further onto center post 80 or, conversely, to move further off of center post 80 depending upon the direction of turning (while still being threaded onto center post 80)(double-arrow 111 shows that T-bar 110 can be turned in either direction). In moving threaded body 108 further onto center post 80, threaded body 108 pushes on one end of first coupling half 76, which causes first coupling half 76 to push on second coupling half 78. Because center post 80 is secured to second coupling half 78 by way of pin 82, this pushing action causes the splits 90 of first and second coupling halves 76, 78 to close and thus causes first and second coupling halves 76, 78 to tighten their grip on bar 42 and short arm 122, respectively. Conversely, turning T-bar 110 in the opposite direction causes first and second coupling halves 76, 78 to loosen their grip on bar 42 and short arm 122, respectively. Pin 82 can be welded to second coupling half 78 (on both sides of pin 82), such as by way of gas tungsten arc welding, 308 or 308L SST, with a filler as needed. This weld area (or the entire post coupling device 14 after assembly) can be passivatedper ATSM A967. Post coupling device 14 is configured for selectively rotating relative to rail post 12 between a plurality of positions, as indicated by double-arrow 118 (a double-arrow, herein, signifying that movement can occur in either direction). That is, when tightener 84 is loosened, post coupling device 14 can be rotated and thus repositioned from one position on bar 42 to another position on bar 42, ridges 92 of post coupling device 14 fitting within grooves 72 of bar 42 when post coupling device 14 is tightened onto bar 42. Further, when tightener 84 is loosened, post coupling device 14 can be raised or lowered on bar 42 (as indicated by double-arrow 117) and then tightened onto bar 42 at a selected elevation on bar 42. Further, first and second coupling halves 76, 78 can be repositioned (for example, by rotation) relative to one another and then brought back together by way of tightener 84 and further prevented from rotating from one another by way of teeth 100; more specifically, when tightener 84 is loosened, second coupling half 78 can be rotated (as indicated by double-arrow 119) relative to first coupling half 76 (this may need to be done in conjunction with T-bar 110 and threaded body 108 so that turning second coupling half 78 does not tighten or loosen post coupling device 14 to an undesired degree), and tightener 84 can be tightened (using T-bar 110) when the desired orientation of second coupling half 78 to first coupling half 76 is achieved. Certain components (not necessarily all) of post coupling device are known.

Fig. 6 shows elevator arm member 16, as well as post coupling device 14 being detached therefrom. Elevator arm member 16, according to one embodiment of the present invention, can include a substantially straight first elongate arm 120 and a substantially straight second elongate arm 122 which is offset from and fixedly coupled with first elongate arm 120. First elongate arm 120 can be longer than second elongate arm 122. Thus, second elongate arm 122 can be referred to as short arm 122, and first elongate arm 120 can be referred to as long arm 120. Elevator arm member 16 can further include an elbow 124. Short arm 122 and long arm 120 can be joined by elbow 124. As shown in Fig. 6, elevator arm member 16 can have an L-shaped configuration formed by short arm 122 and long arm 120. According to one embodiment of the present invention, elevator arm member 16 does not have any other elongate arm other than long and short arms 120, 122; that is, elevator arm member 16 has an absence of an elongate arm in addition to long and short arms 120, 122.

Short arm 122 is configured for being coupled, by way of a post coupling device 14, with rail post 12. Fig. 1 shows that short arm 122 is coupled to rail post 12 by way of post coupling device 14; that is, post coupling device 14 couples short arm 122 with rail post 12. Short arm 122 can be a hollow structure that is substantially cylindrical. Short arm 122 further includes a radially outwardly facing peripheral surface which has a plurality of grooves 126 running longitudinally (that is, axially) on short arm 122. Each groove 126 can be spaced apart from an adjacent groove 126 by eight degrees (this is provided by way of example and not by way of limitation). Each groove 126 is configured to mate with ridges 92 of second coupling half 78 of post coupling device 14 so that short arm 122 is prevented from rotating within first passageway 86 of second coupling half 78 of post coupling device 14. Grooves 126 provide positive engagement while still allowing for adjustment at eight degree increments (thus, grooves 126 can be spaced apart at every eight degrees). Grooves 126 can be formed by a lathe operation, the tool remaining constant while short arm 122 is moving, including rotating every eight degrees so that grooves 126 can be formed). Thus, second coupling half 78 is captured on short arm 122. Short arm 122 can be made of 17-4 PH stainless steel adhering to ASTM A564 or ASTM F899 and can be passivated (per ASTM A967); this is provided by way of example and not by way of limitation. Short arm 122 can include one longitudinal end (that is, the end opposite elbow 124) which is internally threaded which can be predrilled. A stopcap 128 can be externally threaded and can be threadably received by this internally threaded longitudinal end of short arm 122, as indicated by Fig. 6. Stopcap 128 can also be welded to short arm 122 after short arm 122 threadably receives stopcap 128 (and after post coupling device 14 is mounted to short arm 122); this weld can be performed by gas tungsten arc welding, with 17-4 filler, and polished smooth (this is provided by way of example and not by way of limitation). This stopcap 128 (which can have a head, for instance, which is larger in diameter than the threads of endcap 128) can be made of 17-4 PH stainless steel adhering to ASTM A564 or ASTM F899, can be passivated (per ASTM A967), and can be heat treated to condition H900, Rc 40 min; this is provided by way of example and not by way of limitation.

Short arm 122 is configured for selectively rotating relative to post coupling device 14 between a plurality of predetermined positions (determined by the location of grooves 126 of short arm 122) and is configured for selectively moving axially relative to post coupling device 14 between a plurality of positions. Thus, when post coupling device 14 is loosened, short arm 122 can rotate within second coupling half 78 of post coupling device 14 from one position to another position as indicated by double-arrow 130, ridges 92 of second coupling half 78 fitting in grooves 126 of short arm 122 when post coupling device 14 is tightened onto short arm 122. Further, when post coupling device 14 is loosened, short arm 122 can also be moved axially within second coupling half 78 to a desired position, as indicated by double-arrow 132, and post coupling device 14 can be tightened to secure short arm 122 in this other position.

Long arm 120 in general can extend transversely across the bed (not shown) to which rail post 12 is attached. According to one embodiment of the present invention, long arm 120 can be longer than short arm 122. Long arm 120 can be made of 17-4 PH stainless steel adhering to ASTM A564 or ASTM F899 and can be passivated (per ASTM A967); this is provided by way of example and not by way of limitation. Long arm 120 can have one longitudinal end (that is, the free end) which is internally threaded which can be predrilled. An endcap 134 can be externally threaded and thus threadably received by this internally threaded longitudinal end of long arm 120 and also welded to long arm 120. This endcap 134 can be made of 17-4 PH stainless steel adhering to ASTM A564 or ASTM F899 and can be passivated (per ASTM A967); this is provided by way of example and not by way of limitation. The end of long arm 120 to which endcap 134 attaches can have a radiused end feature so that this end of long arm 120 and endcap 134 flow together smoothly with no step. Long arm 120 is attached to short arm 122 (by way of elbow 124) on one longitudinal end of long arm 120, the other longitudinal end of long arm 120 being a free end. Long arm 120 can be a hollow structure or a solid structure. Long arm 120 is coupled to bone hook 22 by way of bone hook coupling device 20; that is, bone hook coupling device 20 couples long arm 120 with bone hook 22. Further, a gun drilling operation, for example, can be used to manufacture the bore through long arm 120. Because of the length of long arm 120, the drill may not be able to drill the entire length of this bore; thus, the bore can be drilled from both sides of long arm 120 and a through-hole mismatch is allowable (a step, to some degree, can be acceptable in this bore through-hole).

Elbow 124 generally has a right angle shape. Elbow 124 includes a shaft 136 projecting in one direction and a blind hole 138 (which can have a flat terminating end) projecting in another direction which is substantially perpendicular to the direction of the shaft 136. Elbow 124 can be made of 17-4 PH stainless steel adhering to ASTM A564 or ASTM F899 and can be passivated (per ASTM A967); this is provided by way of example and not by way of limitation.

During assembly of elevator arm member 16, long arm 120 can be attached to elbow 124, and short arm 122 can be attached to elbow 124. To attach short arm 122 to elbow 124, the following steps can be followed: (1) press fit one longitudinal end (the end which is not threaded) of short arm 122 into blind hole 138 of elbow 124; (2) drill a through-hole through the portion of elbow 124 forming blind hole 138 (on both sides of blind hole 138) and also through the longitudinal end of short arm 122 which has been press fit into blind hole 138 (this drilling can occur after short arm 122 is inserted into elbow 124 and can occur using a milling operation, for example); (3) countersink for weld filler; (4) insert a dowel pin 140 into the drilled hole referenced in step (2)(dowel pin 140 can be made of 416 stainless steel and can be passivated (per ASTM A967); this is provided by way of example and not by way of limitation); and (5) weld both sides of pin 140 to the adjacent sides of elbow 124 (this welding can be performed by gas tungsten arc welding, with 308 or 308L filler as needed, and polished flush). To attach long arm 120 to elbow 124, the following steps can be followed: (1) insert one longitudinal end (the end which is not threaded) of long arm 120 onto shaft 136 of elbow 124; (2) drill a through-hole through this longitudinal end of long arm 120, through shaft 136, and through the other side of this longitudinal end of long arm 120 (this drilling can occur after long arm 120 is inserted onto shaft 136 of elbow 124 and can occur using a milling operation, for example); (3) countersink for weld filler; (4) insert a dowel pin 142 into the drilled hole referenced in step (2)(dowel pin 142 can be made of 416 stainless steel and can be passivated (per ASTM A967); this is provided by way of example and not by way of limitation), dowel pin 142 of long arm 120 being shorter than dowel pin 140 of short arm 122; and (5) weld both sides of pin 142 to the adjacent sides of long arm 120 (this welding can be performed by gas tungsten arc welding, with 17-4 filler as needed, and polished smooth). No shavings or large debris is to be present in either shaft (that is, in long arm 120 and short arm 122). Endcap 134 (associated with long arm 120) is threaded into the threaded longitudinal end of long arm 120; endcap 134 can be welded to long arm 120, such as by laser beam welding, with 17-4 filler as needed, and polished smooth). Stopcap 128 is threaded into the threaded longitudinal end of short arm 122; stopcap 128 can be welded to short arm 122. Short arm 122 and long arm 120 can be respectively welded to elbow 124. For example, the longitudinal edge (adjacent elbow 124) of long arm 120 can be welded to shaft 136 of elbow 124 and/or to the body (which forms blind hole 138) of elbow 124; short arm 122, at about the portion of short arm 122 that is adjacent to the opening of blind hole 138 in elbow 124, can be welded to elbow 124. This welding of short arm 122 and long arm 120 respectively to elbow 124 can be performed by gas tungsten arc welding, with 17-4 filler as needed, and polished smooth (any welding operation described herein is provided by way of example and not by way of limitation). The individual parts of elevator arm member 16, and/or elevator arm member 16 as a whole (as an assembly), can have a satin finish, can be passivated complete per ASTM A967, and can be heat treated to condition H900, Rc 40 min; this is provided by way of example and not by way of limitation.

Further, during assembly of elevator arm member 16 to post coupling device 14, post coupling device 14 is slid onto short arm 122 by way of second coupling half 78 (post coupling device 14 must be oriented as shown in Fig. 6 during this mounting step). Stopcap 128 is threaded into short arm 122 to capture post coupling device 14. Slide post coupling device 14 onto short arm 122 the furthest it can be slid from stopcap 128, and then weld stopcap 128 to short arm 122 (as described above). A function check can be performed such that post coupling device 14 must rotate freely on short arm 122 when the halves 76, 78 of post coupling device 14 are in a relaxed (untightened) position. This weld area (welding stopcap 128 to short arm 122) can be passivated per ASTM A967. According to the embodiment shown in the figures, elevator arm member 16 does not have any other elongate arm other than long and short arms 120, 122.

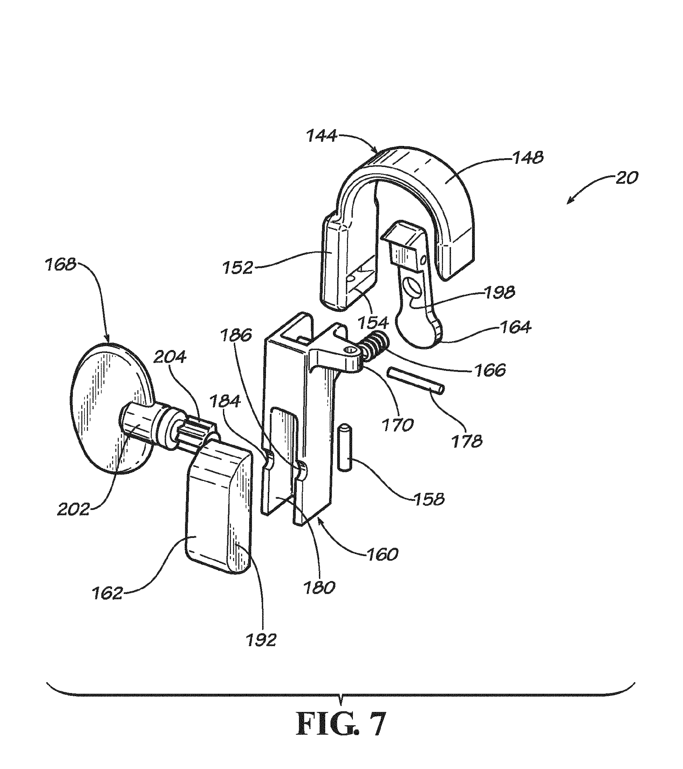

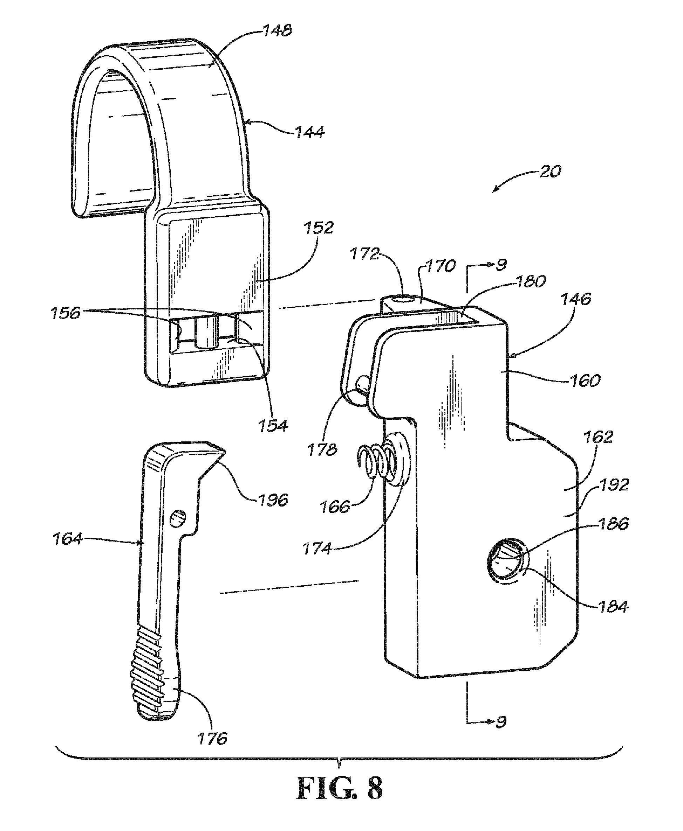

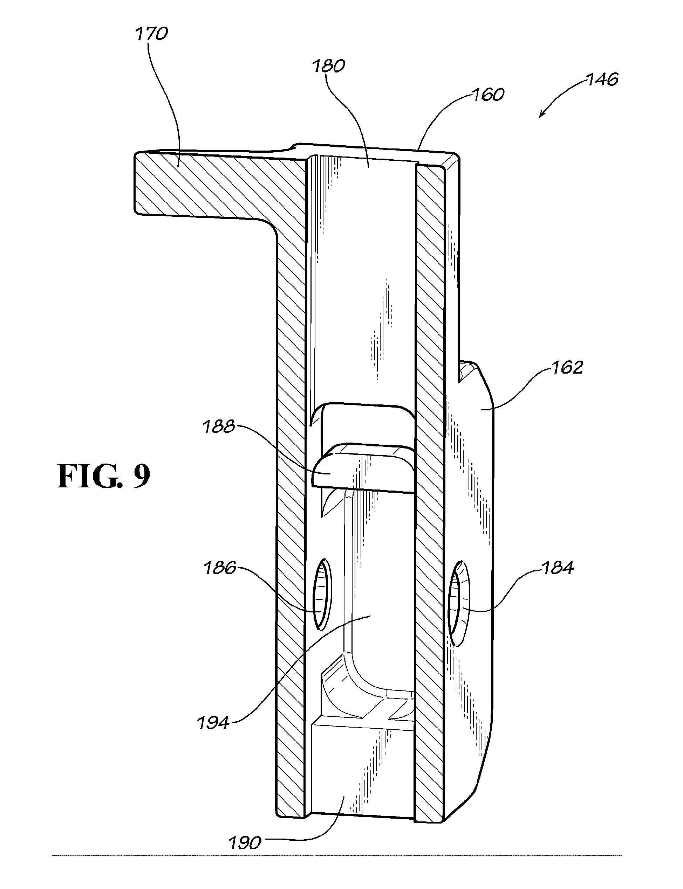

Figs. 7, 8, and 9 show bone hook coupling device 20. Bone hook coupling device 20 couples elevator arm member 16 with bone hook 22. Bone hook coupling device 20 includes an arm hook 144 and a ratchet mechanism 146. Arm hook 144 is pivotally connected to ratchet mechanism 146. When arm hook 144 is placed on long arm 120, arm hook 144 at least partially overlies long arm 120. More specifically, arm hook 144 has an arcuate portion 148 that at least partially overlies long arm 120. Arm hook 144 can be readily placed on long arm 120, readily taken off of long arm 120, and readily moved to different positions transversely along long arm 120. Thus, as shown in Fig. 1, arm hook 144 is not necessarily attached to long arm 120 by any fasteners or anything else other than simply overlying part of the circumference of long arm 120. Arm hook 144 is configured for selectively translating along long arm 120 in a plurality of positions, these positions being chosen by the user (such as the surgeon). That is, a user (such as a surgeon) can slide arm hook 144 in either direction on long arm 120, as indicated by double-arrow 150 in Fig. 1. Thus, arm hook 144 at least partially overlies long arm 120 and is configured for selectively translating along long arm 120 in a plurality of positions.

Arm hook 144 also includes a window section 152 which includes two opposing flat surfaces and a window 154 forming a through-opening extending from one flat face to the other flat face in window section 152. The lateral sides of window can be formed by walls 156 that slope inwardly from each opposing flat face of the window section 152 (that is, these walls 156 slope inwardly from each flat face of window section 152 and can be joined by a substantially flat wall section, the inwardly sloping walls 156 along with the adjoining flat wall section forming a triangular projection inside the window 154 with a generally flat top rather than a pointed top of the triangle). Further, window section 152 of arm hook 144 includes a pivot pin 158 which is positioned in window 154. A bottom portion of window section 152 can include a through-hole, and a top portion of window section 152 can include a corresponding blind hole (which can terminate in a cone-shaped end). During assembly, pivot pin 158 can be inserted through this bottom through-hole and then into blind hole, thereby also traversing window 154. To secure pivot pin 158 to this through-hole and this blind hole, a press fit and/or a weld can be used (or any other suitable connection). Pivot pin 158 can be made of NITRONIC® 60 stainless steel adhering to ASTM A276 or ASTM F899, have a machine finish; this is provided by way of example and not by way of limitation. The remaining portions of arm hook 144 can be made of 17-4 PH stainless steel, ASTM A564, and/or ASTM F899, can have a satin finish, can be passivated (per ASTM A967), and can be heat treated to condition H900, Rc 40 min; this is provided by way of example and not by way of limitation.

Ratchet mechanism 146 is configured for selectively holding bone hook 22 in a plurality of positions. Ratchet mechanism 146 includes a carrier 160, a retaining cap 162, a release trigger 164, a spring 166, and a toothed wheel 168. Carrier 160 of ratchet mechanism 146 includes a projection 170 with a through-hole 172. This through-hole 172 receives pivot pin 158; this reception can be accomplished by inserting projection 170 in window 154, aligning through-hole 172 with the through-hole in the bottom of window section 152 and the blind hole in the top of window section 152 (this through-hole and blind hole of window section communicating with window 154), and inserting pivot pin 158 first through the through-hole in the bottom of window section 152, then through through-hole 172, and then into the blind hole of the top of window section 152 (pivot pin 158 can be welded to window 154). Thus, pivot pin 158 extends through through-hole 172. Projection 170 is thereby configured for pivoting about pivot pin 158. Window 154 forms a pivot boundary relative to projection 170. That is, projection 170 can pivot about pivot pin 158 only so far as the lateral sides (specifically, walls 156, which thus form the pivot boundary) of window 154. Thus, bone hook 22 can pivot twenty-five degrees in each direction from center to allow for femoral shift during elevation or to compensate for an error in initial construct (generally, bone elevator system 10) positioning. The sloped walls 156 of these lateral sides of window 154 allow essentially for a mating contact with the walls 156 of projection 170 as projection 170 swings to either side of window 154. Double-arrow 171 shows that projection 170 can rotate, as bounded by window 154, about pivot pin 158.

Carrier 160 of ratchet mechanism 146 further includes a through-hole in a side wall of carrier 160, this through-hole communicating with a pocket 174 (which can be referred to as a boss) formed on one side of this sidewall; this through-hole helps in flushing pocket 174 and thereby aids in the cleanability of carrier 160. Spring 166 is positioned in pocket 174 on one end of spring 166 and in pocket 198 of release trigger 164 and is thereby captured and held in pocket 174 and pocket 198 by compression on spring 166 provided by carrier 160 and release trigger 164. Spring 166 biases a handle 176 of release trigger 164 away from carrier 160. Carrier 160 includes a pivot bar 178 which fits within a through-hole of an upper portion of release trigger 164 and also in corresponding through-holes in wing projections of carrier 160, release trigger 164 thereby being able to pivot about pivot bar 178. When release trigger 164 is in an engaged position with bone hook 22, handle 176 of release trigger 164 can be either substantially parallel with the side wall (having pocket 174) of carrier or can form an angle with this side wall such that handle is pushed farther away from this sidewall than handle 176 would be if handle 176 were parallel with this side wall of carrier 160. Pivot bar 178 can be welded to carrier in any suitable manner and can be made of, for example, 416 stainless steel; this is provided by way of example and not by way of limitation. Carrier 160 includes a central passageway 180 defined at least in part by the interior of carrier 160. Central passageway 180 receives bone hook 22 therethrough, bone hook 22 being able to move selectively in either direction (shown by double-arrow 182) in passageway 180. Along the open longitudinal side of carrier 160, carrier 160 includes half of a through-hole 184 and half of a pocket 186 opposing the carrier half of the through-hole 184. Carrier 160 can be made of 17-4 PH stainless steel adhering to ASTM A564 or ASTM F899, can have a satin finish (internal surfaces of carrier 160 can be bead blasted), can be passivated (per ASTM A967), and can be heat treated to condition H900, Rc 40 min; this is provided by way of example and not by way of limitation.

Retaining cap 162 is attached to carrier 160, such as by welding. Retaining cap 162 includes an upper projection 188 and a lower projection 190. Retaining cap 162 also includes two opposing sides 192, one side 192 including half of through-hole 184, the other side 192 including half of pocket 186. Retaining cap 162 also includes a chamber 194. Upper and lower projections 188, 190 fit within the open portion of a longitudinal side of carrier 160 so that retaining cap 162 can be joined with carrier 160. Upon bringing retaining cap 162 to carrier 160, the halves of through-hole 184 of carrier 160 and retaining cap 162 mate with one another to form a single through-hole 184. Similarly, the halves of pocket 186 of carrier 160 and retaining cap 162 mate with one another to form a single pocket 186. Retaining cap 162 can be attached to carrier 160 by welding, as described below. Toothed wheel 168 seats within hole 184 and in pocket 186, toothed wheel 168 being captured by carrier 160 and retaining cap 162 and being permitted to rotate within hole 184 and pocket 186. When retaining cap 162 is attached to carrier 160, chamber 194 forms a side chamber to the side of, and communicating with, passageway 180. Retaining cap 162 can be made of 17-4 PH stainless steel adhering to ASTM A564 or ASTM F899, can have a satin finish, can be passivated (per ASTM A967), and can be heat treated to condition H900, Rc 40 min, and internal surfaces of carrier 160 can be bead blasted; this is provided by way of example and not by way of limitation.

Release trigger 164 can also be referred to as a lever. Release trigger 164 includes handle 176 and a tooth 196 on an upper portion of release trigger 164. An exterior surface of handle 176 of release trigger 164 can include a plurality of ridges (as shown) to facilitate the gripping or frictional engagement of handle by the thumb or finger of a surgeon; these ridges can have grooves therebetween which have a radius or which individually come to a sharp point. An inner surface of handle can include a shallow pocket 198 which receives one end of spring 166, spring 166 thereby being held by this pocket 198 on one end of spring 166 and being held by pocket 174 of carrier 160 on the other end of spring 166 and biasing handle 176 away from carrier 160 and thereby biasing tooth 196 of release trigger 164 towards bone hook 22. Release trigger 164 pivots about pivot bar 178 of carrier 168 by way of a through-hole through the top portion of release trigger 164. When handle 176 is in a first position pushed away from carrier 160 by spring 166, tooth 196 is in an engagement position so as to engage with a groove between teeth 200 of bone hook 22. When handle 176 is in a second position pressed towards carrier 160 by a surgeon, tooth 196 is in a disengagement position so as not to engage bone hook 22. Thus, when handle 176 is released by a surgeon, release trigger 164 returns to a home position, that being the engagement position. Release trigger 164 can be made of 455 stainless steel adhering to ASTM F899 and can be heat treated to Rc 48-52; this is provided by way of example and not by way of limitation. All surfaces except the carrier facing surface of release trigger 164 (this carrier facing surface being opposing the thumb gripping ridges of release trigger 164, this carrier facing surface extending from the inwardly facing bottom corner of release trigger 164, past pocket 198, and up to the tip of tooth 196) release trigger 164 can have a high sheen finish (near buff), and this carrier facing surface of release trigger 164 can have a satin finish. Release trigger 164 can be passivated (per ASTM A967). Release trigger 164 can be gold plated using Electro-spec. That is, release trigger 164 can be plated with gold using an electrical adhesion process. A base coat with a non-nickel alloyed Technic acid gold strike can be used. An outer coat with orosene 990 HS per ASTM B488-01 code C can be used. The width of release trigger 164 (this width being taken above the curved portion defining the thumb/finger-depressing portion of release trigger 164 and running parallel to the ridges of the thumb/finger-depressing portion of release trigger 164) and the diameter of the through-hole in release trigger 164 which receives pivot bar 178 can be maintained after coating.

Toothed wheel 168 includes a shaft 202, a plurality of teeth 204, and a tab 206. Shaft 202 (which can be referred to as a pinion shaft) can include a groove on one end that receives tab 206 and is welded to tab 206 (which can be referred to as a thumb piece 206). In one embodiment, tab 206 can have be a generally flat structure having an oval or elliptical cross-section. Tab 206 can have a groove formed in a side edge of the flat structure of tab 206, the groove having a generally straight side and seating within the groove of shaft 202, the groove of tab 206 optionally having a female dovetail in the inner corners. Tab 206 is used to turn toothed wheel 168 by a surgeon. Tab 206 can be made of 304 stainless steel adhering to ASTM F899 and can have a satin finish; this is provided by way of example and not by way of limitation. Further, in one embodiment, shaft 202 and teeth 204 can be formed integral with one another (that is, they can be formed from the same piece). Alternatively, shaft 202 and teeth 204 can be formed separately and joined together to form a unitary assembly. According to either alternative, shaft 202 can be positioned in through-hole 184, in side chamber 194, and in pocket 186. Further, the plurality of teeth 204 can have a hub from which the teeth 204 extend. Shaft 202 can extend through this hub (shaft 202 and this hub thus being connected by welding) or, alternatively, can be formed in one piece with this hub. Each tooth 204 can be flat at its crest (at the distal end of the tooth 204). Shaft 202 and teeth 204 can be made of 455 stainless steel adhering to ASTM F899 and can have a satin finish; this is provided by way of example and not by way of limitation. Tab 206 can be fully seated in the groove of shaft 202. Tab 206 can be welded to shaft 202 using laser beam welding (such as 308 or 308 L, with filler as needed, and polished smooth) such as where the tab groove contacts the groove of shaft 202, such as (but not limited to) the base of the shaft groove. Further, the toothed wheel 168 (the assembly) can be heat treated (to Rc 48-52) to achieve the specified hardness for the structure formed of shaft 202 and teeth 204. Further, toothed wheel 168 (the assembly) can be passivated complete (per ASTM A967) after welding and heat treating; this is provided by way of example and not by way of limitation.

During assembly, toothed wheel 168 is captured with carrier 160 and retaining cap 162. That is, shaft 202 can be positioned against carrier 160 by half portions of through-hole 184 and pocket 186, and then retaining cap 162 can be attached to carrier 160 to form the other half of through-hole 184 and pocket 186, shaft 202 and teeth 204 being held thereby and being able to pivot within through-hole 184 and pocket 186. Toothed wheel 168 is supported in side chamber 194 by through-hole 184 and pocket 186, shaft 202 of toothed wheel 168 being supported in through-hole 184, and distal end of shaft 202 being supported in pocket 186. Fig. 7 shows that the toothed portion 204 of toothed wheel 168 is positioned at least partially in side chamber 194, a portion of one or more teeth 204 projecting in central passageway 180 so as to engage with the teeth 200 of bone hook 22. Retaining cap 162 is welded to carrier 160 (after capturing toothed wheel 168 with carrier 160 and retaining cap 162), such as by way of micro-TIG (tungsten inert gas welding), with 17-4 filler, relative to the entire seam, and polished smooth. This includes that the bottom of carrier 160 and the bottom of retaining cap 162 (the bottom being opposite the top of carrier 160 which is closer to the arcuate section 148 of the arm hook 144) are welded to one another using micro-TIG, with 17-4 filler as needed, and polished smooth. A function check can be performed with LMC and MMC gauge, checking to ensure, for example, geometry has been maintained. Further, the bottom of carrier walls of carrier 160 can be welded to the bottom of retaining cap 162 and to the bottom of lower projection 190 of retaining cap 162, such as by way of micro-TIG, with 17-4 filler as needed, and polished flush. The internal geometry must be maintained while welding the end seam (for example, the bottom of carrier walls of carrier 160 that weld to the bottom of retaining cap 162 and to the bottom of lower projection 190 of retaining cap 162), and a function check can be done with LMC and MMC gauges. Spring 166 is captured with release trigger 164 and carrier 160. Release trigger 164 is cross-pinned to carrier 160 with pin 178, and both sides of pin 178 are welded, such as by way of laser beam welding, 17-4 filler, and polished flush. Arm hook 144 is captured with pin 158 and carrier 160; arm hook 144 is cross-pinned to carrier 160 with pin 158, and both sides of pin 158 (or just the bottom of pin 158 to the bottom of window section 152 opposite the arcuate section 148) can be welded to arm hook 144, such as by way of gas tungsten arc welding, with 17-4 filler, and polished flush. Further, the weld areas of bone hook coupling device 20 can be passivated per ASTM A967. A Week lube (lubricant) or an equivalent can be applied to the bone hook coupling device 20 (the assembly). Toothed wheel 168 must rotate freely. A function check of the bone hook coupling device 20 (the assembly) can be performed with LMC and MMC gauge, for example, to ensure proper dimensions associated with each of the welding operations.

Fig. 1 shows bone hook 22. Bone hook 22 can be a substantially rigid structure. Bone hook 22 includes a hook 208 and a longitudinally extending shaft 210 with a plurality of teeth 200 thereon. Bone hook 22 can further include a stop 212 which prevents bone hook 22 from being inserted too far into carrier of ratchet mechanism 146; that is, stop 212 provides a positive stop on bone hook 22 to prevent ratcheting too far. Hook 208 can be selectively provided with a certain radius in the curve of hook 208, this curve being shown in Fig. 1. Fig. 1 shows the curve of hook 208 being provided with a certain radius, but it is understood that hook 208 can be provided with a curve having a smaller or a larger radius, depending upon the application. Further, bone hook 22 can be provided as a part of a kit associated with bone elevator system 10 (all or, alternatively, certain ones of bone elevator system 10 can be provided as part of the kit); according to one embodiment of this kit, the kit can include a two bone hooks 22 each with a curve having a different radius relative to one another. The shaft 210 of bone hook 22 can be inserted through the bottom of carrier 160 of ratchet mechanism 146. When release trigger 164 is in an engagement position, release trigger 164 can selectively engage a particular groove between bone hook teeth 200 so as to lock bone hook 22 in a predetermined position relative to ratchet mechanism 146. The predetermined positions are determined by the grooves between bone hook teeth 200. In this way, ratchet mechanism 146 is configured for selectively holding bone hook 22 in a plurality of predetermined positions. Further, toothed wheel 168 can be turned to selectively move bone hook 22 upwardly (moving hook 208 closer to carrier 160 of ratchet mechanism 146) relative to ratchet mechanism 146, teeth 204 of toothed wheel 168 and teeth 200 of bone hook 22 being angled so as to engage one another and to allow teeth 204 of toothed wheel 168 to grab and thereby ratchet bone hook 22 upwardly. Release trigger 164 then prevents bone hook 22 from falling back through central passageway 180 by the force of gravity after releasing toothed wheel 168. When release trigger 164 is unlocked from bone hook 22, bone hook 22 can freely fall through central passageway 180 (absent prevention by a surgeon or operating room personnel). Bone hook 22 can be made of 17-4 PH stainless steel adhering to ASTM F899 or ASTM A564, can have a satin finish, can be passivated (per ASTM A967), and can be heat treated to condition H900, Rc 40 min; this is provided by way of example and not by way of limitation.

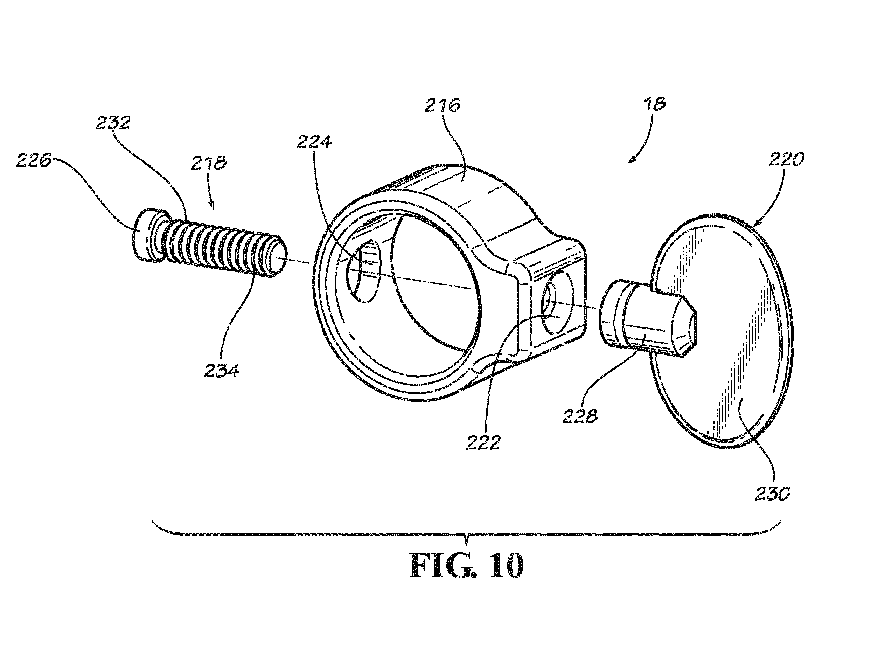

Bone elevator system 10 can optionally include a lateral retention ring assembly 18 (which can also be referred to as a collar stop), which is shown in Fig. 10. Lateral retention ring assembly 18 can be added to prevent medial or lateral shift of bone hook 22. Lateral retention ring assembly 18 includes a collar 216 (which can also be referred to as the main body), a threaded post 218, and a cap assembly 220. Collar 216 is formed as an annular ring. Collar 216 includes a threaded through-hole 222 and an additional opposing through-hole 224. The threads of this threaded through-hole 222 may extend only through a portion of the thickness of this part of the ring 216. Collar 216 can be made of 17-4 PH stainless steel adhering to ASTM F899 or ASTM A564, can have a satin finish, can be passivated (per ASTM A967), and can be heat treated to condition H900, Rc 40 min; this is provided by way of example and not by way of limitation. Threaded post 218 includes a bottom stopper 226 which is configured for engaging long arm 120 when threaded post 218 is extended far enough to engage long arm 120. Threaded post 218 further includes a bottom threaded portion 232 (near the bottom stopper) and an upper threaded section 234. Bottom threaded portion 232 threadably engages the threads of the threaded through-hole 222 of collar 216 so as to attach threaded post 219 to collar 216. The upper threaded portion 234 threadably engages the threads of cap 228 of cap assembly 220 and thereby attaches threaded post 218 to cap 228. Threaded post 218 can be made of 304 stainless steel adhering to ASTM F899, can have a satin finish, and can be passivated (per ASTM A967); this is provided by way of example and not by way of limitation. Cap assembly 220 includes cap 228 and a tab 230 (which can also be referred to as a thumb piece 230). Cap 228 includes internal threads and a groove which is adjacent the internal threads. The internal threads of cap 228 threadably receive the upper threaded portion 234 of threaded post 218. Cap 228 can be made of 304 stainless steel adhering to ASTM F899, can have a satin finish (excluding the internal threads of cap), and can be passivated (per ASTM A967); this is provided by way of example and not by way of limitation. Tab 230 is like the tab 206 of ratchet mechanism 146 and includes a groove which is positioned in groove of cap 228. This groove of tab 230 can include female dovetails at the corners of the groove. Tab 230 can be made of 304 stainless steel adhering to ASTM F899 and can have a satin finish; this is provided by way of example and not by way of limitation. Tab 230 is fully seated into the groove of cap 228. Tab 230 can be welded to cap 228 using laser beam welding (such as 308 or 308 L, with filler as needed, and polished smooth) such as where the groove of tab 230 contacts the groove of cap 228 (cap 228 can also be referred to as a shaft), such as (but not limited to) the base of the groove of cap 228. Cap assembly 220 can be passivated per ASTM A967. During assembly of lateral retention ring assembly 18, threaded post 218 is inserted through the hole in collar 216 opposing the threaded through-hole 222 and then into the threaded through-hole 222 until threaded post 218 stops. Cap 228 is then threaded onto threaded post 218 until cap 228 is fully seated. Then, cap 228 is welded to threaded post 218 (such as at the base of cap 228 facing collar 216), such as by way of gas tungsten arc welding (micro-TIG (tungsten inert gas welding) is allowable), 308 or 308L, filler as needed, and polished smooth. This weld area can occur in the area of threaded post 218 between the threaded sections 232, 234 but can include a portion of the threads as well. This weld area can be passivated per ASTM A967. In use, with threaded post 218 in a position in which it will not engage with long arm 120, collar 216 is slid onto the free end of long arm 120 to a selected position on long arm 120. After collar 216 is slid to the desired point on long arm 120, threaded post 218 is turned (by way of tab 230) in a direction so that threaded post 218 extends further towards long arm 120 and the bottom portion 232 of threaded post 218 presses against long arm 120, thereby causing lateral retention ring assembly 18 to be tightened and fixedly engaged with long arm 120. In this way, lateral retention ring assembly 18 keeps ratchet mechanism 146 from moving at least in one direction along long arm 120. Lateral retention ring assembly 18 can be slid on long arm 120 in either direction, as indicated by double-arrow 238. Two such lateral retention ring assemblies 18 can be used to capture bone hook coupling device 20 therebetween, if so desired. Only one such lateral retention ring assembly 18 is shown in the figures.

During assembly, rail post 12 can be placed on a horizontal rail 24 and secured thereto. Post coupling device 14 can be placed onto short arm 122 of elevator arm member 16 and then (or vice versa) onto rail post 12. Arm hook 144 can be placed onto long arm 120, and lateral retention ring assembly 18 can be selectively positioned on and secured to long arm 120. The straight end of bone hook 22 can be inserted up through the central passageway 180 of carrier 160 of ratchet mechanism 146 and secured to carrier 160 by way of release trigger 164.

In use, bone elevator system 10 can optionally be mounted to a standard operating table. Bone elevator system 10 can be oriented in a variety of positions. Rail post 12 can be slid along rail 24 and clamped thereto in various positions (as indicated by double-arrow 26). Further, first coupling half 76 of post coupling device 14 can be raised or lowered on bar 42 (as indicated by double-arrow 117) and can be rotated in either direction on bar 42 (as indicated by double-arrowl 18) and secured thereto at a desired position. Further, first and second coupling halves 76, 78 can also be rotated relative to one another (as indicated by double arrow 119). For instance, second coupling half 78, when tightener 84 is loosened, can be rotated in either direction relative to first coupling half 76 and then secured back to first coupling half 76 by way of tightener 84; in this way, short bar 122 (and thus elevator arm member 16) can be angled upwardly or downwardly at a selected angle. Further, short bar 122 can be slid axially relative to second coupling half 78 so that second coupling half 78 is positioned closer or farther from elbow 124, for instance (as indicated by double-arrow 132). Further, short arm 122 can rotate within second coupling half 78 and secured thereto by tightener 84 (as indicated by double-arrow 130). While not limited to this position, short arm 122 generally runs parallel to the longitudinal extent of a patient table or bed, and long arm generally extends transversely relative to the patient table or bed. Further, by way of arm hook 144, bone hook coupling device 20 can be translated along long arm 120 (as indicated by double-arrow 150) and positioned at virtually any location along long arm 120 (and arm hook 144 can be rotated to some degree on long arm 120, as indicated by double-arrow 236), and lateral retention ring assembly 18 can be used to help secure bone hook coupling device 20 on long arm 120 (lateral retention ring assembly 18 being translatable along long arm 120, as indicated by double-arrow 238). Further, by way of projection 170, ratchet mechanism 146 (which holds bone hook 22) can pivot on pivot pin 158 (for example, in a fifty degree swath)(as indicated by double-arrow 171). Further, arm hook 144 can pivot to some degree about long arm 120 (as indicated by double-arrow 236). Further, bone hook 22 can be raised or lowered relative to ratchet mechanism 146 and held thereby (as indicated by double-arrow 182). Toothed wheel 168 can be turned (as indicated by double-arrow 214) to raise or lower bone hook 22. These ways of moving and orienting bone elevator system 10 allows the surgeon to selectively position bone hook 22.

Further, the following generally describes a surgical procedure using the bone elevator system 10 of the present invention; it is understood, however, that a surgeon is not limited to this description of a surgical procedure. The femoral head is cut off from the femur; this can occur after the femur is dislocated from the acetabulum, or, the femoral head can first be cut off and then the femoral head can be removed from the acetabulum. After the femoral head is cut off, bone hook 22 is placed under the femur (bone hook 22 can already be located in ratchet mechanism 146), bone hook system 10 having already been positioned in the desired position relative to the femur. Bone hook system 10 can be repositioned as desired prior to elevating the femur. The femur is then elevated with bone hook 22 (using ratchet mechanism 146), without straining the patient's muscles. Bone hook 22 thus elevates the femur and then holds the femur in a desired position. The femur is then broached to prepare the femur to receive a femoral implant (an acetabulum implant can be inserted before the femur is broached). At different points during the surgical procedure, the femur can be manually manipulated to achieve the proper orientation.

The components of the bone elevator system 10 of the present invention can be made by a variety of manufacturing processes. For example, the manufacturing processes used may include machining (such as wire EDM), lathe operations, milling operations (such as 3, 4, or 5 axis milling operations), TIG welding, and/or micro-TIG welding.

The present invention further provides a method for using a bone elevator system. The method includes: providing a bone hook 22, a first coupling device 20, and an elevator arm member 16 including a substantially straight first elongate arm 120 and a substantially straight second elongate arm 122 which is offset from and fixedly coupled with first elongate arm 120; coupling, using first coupling device 20, first elongate arm 120 with bone hook 22; coupling, using second coupling device 14, second elongate arm 122 with an anchoring post 12. First coupling device 20 includes a ratchet mechanism 146 and an arm hook 144 pivotally connected to ratchet mechanism 146, the method further including holding selectively, using ratchet mechanism 146, bone hook 22 in a plurality of positions, arm hook 144 at least partially overlying first elongate arm 120, the method further including translating selectively arm hook 144 along first elongate arm 120 in a plurality of positions, anchoring post 12 being a rail post 12 which is coupled with a rail 24. Arm hook 144 includes a window 154 and a pivot pin 158 in window 154, ratchet mechanism 146 including a projection 170 with a through-hole 172, pivot pin 158 extending through through-hole 172, the method including pivoting projection 170 about pivot pin 158, window 154 forming a pivot boundary 154 relative to projection 170. Elevator arm member 16 has an L-shaped configuration formed by first elongate arm 120 and second elongate arm 122. The method can further include rotating selectively second elongate arm 122 relative to second coupling device 14 between a plurality of predetermined positions and moving selectively second elongate arm 122 axially relative to second coupling device 14 between a plurality of positions. The method can further include rotating selectively second coupling device 14 relative to rail post 12 between a plurality of positions. Elevator arm member 16 has an absence of an elongate arm in addition to first elongate arm 120 and second elongate arm 122, first elongate arm 120 being longer than second elongate arm 122.

While this invention has been described with respect to at least one embodiment, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

The invention comprises aspects which are disclosed in the sentences below, which are part of the description but not claims in accordance with J 15/88 of the Boards of appeal

### Sentences

1. A bone elevator system, comprising:
   a bone hook;
   a first coupling device; and
   an elevator arm member including a substantially straight first elongate arm and a substantially straight second elongate arm which is offset from and fixedly coupled with said first elongate arm, said first coupling device coupling said first elongate arm with said bone hook, said second elongate arm configured for being coupled, by way of a second coupling device, with an anchoring post.
2. The bone elevator system of sentence 1, further including said second coupling device and said anchoring post, said anchoring post being a rail post, said second coupling device coupling said second elongate arm with said rail post, said rail post being configured for coupling with a rail.
3. The bone elevator system of sentence 2, wherein said first coupling device includes a ratchet mechanism and an arm hook pivotally connected to said ratchet mechanism, said ratchet mechanism configured for selectively holding said bone hook in a plurality of positions, said arm hook at least partially overlying said first elongate arm and being configured for selectively translating along said first elongate arm in a plurality of positions.
4. The bone elevator system of sentence 3, wherein said arm hook includes a window and a pivot pin in said window, said ratchet mechanism including a projection with a through-hole, said pivot pin extending through said through-hole, said projection being configured for pivoting about said pivot pin, said window forming a pivot boundary relative to said projection.
5. The bone elevator system of sentence 4, wherein said elevator arm member has an L-shaped configuration formed by said first elongate arm and said second elongate arm.
6. The bone elevator system of sentence 5, wherein said second elongate arm is configured for selectively rotating relative to said second coupling device between a plurality of predetermined positions and is configured for selectively moving axially relative to said second coupling device between a plurality of positions.
7. The bone elevator system of sentence 6, wherein said second coupling device is configured for selectively rotating relative to said rail post between a plurality of positions.
8. The bone elevator system of sentence 7, wherein said elevator arm member has an absence of an elongate arm in addition to said first elongate arm and said second elongate arm, said first elongate arm being longer than said second elongate arm.
9. A method for using a bone elevator system, said method comprising the steps of:
   providing a bone hook, a first coupling device, and an elevator arm member including a substantially straight first elongate arm and a substantially straight second elongate arm which is offset from and fixedly coupled with said first elongate arm;
   coupling, using said first coupling device, said first elongate arm with said bone hook;
   coupling, using said second coupling device, said second elongate arm with an anchoring post.
10. The method of sentence 9, wherein said first coupling device includes a ratchet mechanism and an arm hook pivotally connected to said ratchet mechanism, the method further including holding selectively, using said ratchet mechanism, said bone hook in a plurality of positions, said arm hook at least partially overlying said first elongate arm, the method further including translating selectively said arm hook along said first elongate arm in a plurality of positions, said anchoring post being a rail post which is coupled with a rail.
11. The method of sentence 10, wherein said arm hook includes a window and a pivot pin in said window, said ratchet mechanism including a projection with a through-hole, said pivot pin extending through said through-hole, the method including pivoting said projection about said pivot pin, said window forming a pivot boundary relative to said projection.
12. The method of sentence 11, wherein said elevator arm member has an L-shaped configuration formed by said first elongate arm and said second elongate arm.
13. The method of sentence 12, further including rotating selectively said second elongate arm relative to said second coupling device between a plurality of predetermined positions and moving selectively said second elongate arm axially relative to said second coupling device between a plurality of positions.
14. The method of sentence 13, further including rotating selectively said second coupling device relative to said rail post between a plurality of positions.
15. The method of sentence 14, wherein said elevator arm member has an absence of an elongate arm in addition to said first elongate arm and said second elongate arm, said first elongate arm being longer than said second elongate arm.

## Claims

1. A bone elevator system, comprising:
a bone hook;
a first coupling device; and
an elevator arm member including a substantially straight first elongate arm and a substantially straight second elongate arm which is offset from and fixedly coupled with said first elongate arm, said first coupling device coupling said first elongate arm with said bone hook, said second elongate arm configured for being coupled, by way of a second coupling device, with an anchoring post.

2. The bone elevator system of claim 1, further including said second coupling device and said anchoring post, said anchoring post being a rail post, said second coupling device coupling said second elongate arm with said rail post, said rail post being configured for coupling with a rail.

3. The bone elevator system of at least one of the claims 1 to 2, wherein said first coupling device includes a ratchet mechanism and an arm hook pivotally connected to said ratchet mechanism, said ratchet mechanism configured for selectively holding said bone hook in a plurality of positions, said arm hook at least partially overlying said first elongate arm and being configured for selectively translating along said first elongate arm in a plurality of positions.

4. The bone elevator system of claim 3, wherein said arm hook includes a window and a pivot pin in said window, said ratchet mechanism including a projection with a through-hole, said pivot pin extending through said through-hole, said projection being configured for pivoting about said pivot pin, said window forming a pivot boundary relative to said projection.

5. The bone elevator system of at least one of the claims 1 to 4, wherein said elevator arm member has an L-shaped configuration formed by said first elongate arm and said second elongate arm.

6. The bone elevator system of claim 5, wherein said second elongate arm is configured for selectively rotating relative to said second coupling device between a plurality of predetermined positions and is configured for selectively moving axially relative to said second coupling device between a plurality of positions.

7. The bone elevator system of at least one of the claims 1 to 6, wherein said second coupling device is configured for selectively rotating relative to said rail post between a plurality of positions.

8. The bone elevator system of at least one of the claims 1 to 7, wherein said elevator arm member has an absence of an elongate arm in addition to said first elongate arm and said second elongate arm, said first elongate arm being longer than said second elongate arm.

9. A method for using a bone elevator system, said method comprising the steps of:
providing a bone hook, a first coupling device, and an elevator arm member including a substantially straight first elongate arm and a substantially straight second elongate arm which is offset from and fixedly coupled with said first elongate arm;
coupling, using said first coupling device, said first elongate arm with said bone hook;
coupling, using said second coupling device, said second elongate arm with an anchoring post.

10. The method of claim 9, wherein said first coupling device includes a ratchet mechanism and an arm hook pivotally connected to said ratchet mechanism, the method further including holding selectively, using said ratchet mechanism, said bone hook in a plurality of positions, said arm hook at least partially overlying said first elongate arm, the method further including translating selectively said arm hook along said first elongate arm in a plurality of positions, said anchoring post being a rail post which is coupled with a rail.

11. The method of claim 10, wherein said arm hook includes a window and a pivot pin in said window, said ratchet mechanism including a projection with a through-hole, said pivot pin extending through said through-hole, the method including pivoting said projection about said pivot pin, said window forming a pivot boundary relative to said projection.

12. The method of at least one of the claims 9 to 11, wherein said elevator arm member has an L-shaped configuration formed by said first elongate arm and said second elongate arm.

13. The method of at least one of the claims 9 to 12, further including rotating selectively said second elongate arm relative to said second coupling device between a plurality of predetermined positions and moving selectively said second elongate arm axially relative to said second coupling device between a plurality of positions.

14. The method of at least one of the claims 9 to 13, further including rotating selectively said second coupling device relative to said rail post between a plurality of positions.

15. The method of at least one of the claims 9 to 14, wherein said elevator arm member has an absence of an elongate arm in addition to said first elongate arm and said second elongate arm, said first elongate arm being longer than said second elongate arm.
